(19) Europäisches Patentamt
European Patent Office
Office européen des brevets

(11) **EP 4 684 783 A1**

(12) **EUROPEAN PATENT APPLICATION**
published in accordance with Art. 153(4) EPC

(43) Date of publication:
**28.01.2026 Bulletin 2026/05**

(21) Application number: 24865931.0

(22) Date of filing: **20.08.2024**

(51) International Patent Classification (IPC):
*A61K 31/19* (2006.01)    *A61P 17/14* (2006.01)
*A61K 8/365* (2006.01)    *A61Q 7/00* (2006.01)
*A61Q 5/00* (2006.01)    *A61K 31/191* (2006.01)
*A61Q 9/04* (2006.01)

(52) Cooperative Patent Classification (CPC):
A61K 8/365; A61K 31/19; A61K 31/191;
A61P 17/14; A61Q 5/00; A61Q 7/00; A61Q 9/04

(86) International application number:
**PCT/KR2024/096040**

(87) International publication number:
**WO 2025/058492 (20.03.2025 Gazette 2025/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.09.2023 KR 20230120959
26.07.2024 KR 20240099301
26.07.2024 KR 20240099302**

(71) Applicant: **LG Chem, Ltd.
Seoul 07336 (KR)**

(72) Inventors:
• **PARK, Hun**
  **Daejeon 34122 (KR)**
• **JUNG, Woochul**
  **Daejeon 34122 (KR)**
• **YANG, Jungil**
  **Daejeon 34122 (KR)**
• **KANG, Donggyun**
  **Daejeon 34122 (KR)**

(74) Representative: **Plasseraud IP
104 Rue de Richelieu
CS92104
75080 Paris Cedex 02 (FR)**

(54) **COSMETIC COMPOSITION FOR PREVENTING, ALLEVIATING, OR AMELIORATING HAIR LOSS**

(57) The present disclosure relates to a cosmetic composition for preventing, alleviating or ameliorating alopecia. According to the present disclosure, a cosmetic composition that not only inhibits the action of TGF-β1 factor, but also has an inhibitory effect on 5α-reductase, and therefore can exhibit an excellent effect for preventing, alleviating, or ameliorating alopecia is provided.

【FIG. 1】

**Description**

**[TECHNICAL FIELD]**

CROSS-REFERENCE TO RELATED APPLICATION(S)

This application claims priority to and the benefit of Korean Patent Application No. 10-2023-0120959, filed on September 12, 2023, Korean Patent Application No. 10-2024-0099301, filed on July 26, 2024, and Korean Patent Application No. 10-2024-0099302, filed on July 26, 2024, with the Korean Intellectual Property Office, the disclosures of which are incorporated herein by reference in their entirety.

The present disclosure relates to a cosmetic composition exhibiting the effect of preventing, alleviating or ameliorating alopecia.

**[BACKGROUND OF ART]**

Alopecia appears due to a variety of factors, including genetics, disease, childbirth, stress, hormones, excess sebum secretion, poor blood circulation, decreased scalp function, and aging.

In general, alopecia refers to a condition in which the ratio of hair in the anagen decreases in the hair growth cycle, and the ratio of hair in the catagen or telogen increases, resulting in an increase in the number of hairs that fall out abnormally. As alopecia progresses, the period of the anagen becomes shorter, the transition to the catagen and telogen is promoted, and the volume of the follicle dermal papilla decreases, so that the hair follicle become increasingly smaller. Therefore, in order to treat alopecia, it is important to allow the hair follicles in the telogen to quickly transition to the anagen and to extend the shortened anagen. In addition, it is important to inhibit the action of TGF-$\beta$1, which is a factor that promotes the anagen of hair follicles to the catagen.

Meanwhile, male pattern alopecia, which accounts for the highest percentage of hair loss patients, is also called androgenetic alopecia. Androgenetic alopecia occurs when dihydrotestosterone(DHT), which is generated by the binding of the male hormone testosterone and its active enzyme 5$\alpha$-reductase, acts on the hair follicle to inhibit cell division and inhibit hair growth.

Commercially available alopecia-related products generally contain effective ingredients that are effective in strengthening hair root function, moisturizing the scalp, and promoting blood circulation. However, it is difficult to find a product that shows a clear effect on hair loss symptoms. In addition, most 5$\alpha$-reductase inhibitors are steroidal ingredients, which have the problem of inducing various side effects.

**[DETAILED DESCRIPTION OF THE INVENTION]**

**[Technical Problem]**

It is an object of the present disclosure to provide a cosmetic composition for preventing, alleviating or ameliorating alopecia, comprising a non-steroidal compound as an effective ingredient.

It is another object of the present disclosure to provide a pharmaceutical composition for preventing or treating alopecia, comprising a non-steroidal compound as an effective ingredient.

**[Technical Solution]**

According to an embodiment of the present disclosure, there is provided a cosmetic composition for preventing, alleviating or ameliorating alopecia, comprising 3-hydroxypropionic acid or a derivative thereof as an effective ingredient.

According to another embodiment of the present disclosure, there is provided use of 3-hydroxypropionic acid or a derivative thereof for the preparation of cosmetics for preventing, alleviating or ameliorating alopecia.

According to yet another embodiment of the present disclosure, there is provided a method of preventing, alleviating or ameliorating alopecia by using a cosmetic composition comprising 3-hydroxypropionic acid or a derivative thereof as an active ingredient.

According to yet another embodiment of the present disclosure, there is provided a pharmaceutical composition for preventing or treating alopecia comprising 3-hydroxypropionic acid or a derivative thereof as an effective ingredient.

According to yet another embodiment of the present disclosure, there is provided use of 3-hydroxypropionic acid or a derivative thereof for the preparation of medicines for preventing or treating alopecia.

According to yet another embodiment of the present disclosure, there is provided a method of preventing, alleviating or treating alopecia by using a pharmaceutical composition comprising 3-hydroxypropionic acid or a derivative thereof as an active ingredient.

Hereinafter, a cosmetic composition for preventing, alleviating or ameliorating alopecia or a pharmaceutical composition for preventing or treating alopecia according to specific embodiments of the present disclosure will be described in more detail.

Terms or words used in the present specification and claims should not be construed as limited to ordinary or dictionary terms, and the present disclosure should be construed with meanings and concepts that are consistent with the technical idea of the present disclosure based on the principle that the inventors may appropriately define concepts of the terms to appropriately describe their own invention in the best way.

Unless otherwise defined, all technical terms and scientific terms used in the specification have the general meaning understood by those skilled in the art to which the present disclosure belongs. The terms used in the specification are only to effectively describe a specific embodiment, but are not intended to limit the present disclosure.

While the present disclosure may be modified in various ways and take on various alternative forms, specific embodiments thereof are described in detail below. However, it should be understood that there is no intent to limit the present disclosure to the particular forms disclosed, but on the contrary, the present disclosure covers all modifications, equivalents, and alternatives falling within the spirit and scope of the present disclosure.

Singular terms used in the specification include plural terms, unless the context clearly indicates otherwise.

The terms "comprise," "include", "have", etc. are used herein to specify the presence of stated features, numbers, regions, steps, operations, elements, and/or components, but do not preclude the presence or addition of one or more other features, regions, numbers, steps, operations, elements, components, and/or groups thereof.

In describing temporal relationships, for example, when the temporal order is described using "after", "subsequent", "next", or "before", the case of a noncontinuous relationship may be included, unless "just" or "directly" is used.

As used herein, the term "hair loss" or "alopecia" means a condition in which hair is absent in areas where hair should be normally present due to abnormal hair loss.

As used herein, the term "prevention" means inhibiting the occurrence of symptoms caused by alopecia or symptoms caused by complications of alopecia. As used herein, the term "alleviation" means the reduction of symptoms caused by alopecia. As used herein, the term "amelioration" means the improvement of symptoms caused by alopecia or symptoms caused by complications of alopecia.

As used herein, the term "treatment" means alleviating or eliminating symptoms caused by alopecia or symptoms caused by complications of alopecia.

As used herein, the term "derivative(s)" means a compound that has been modified from their parent organic compound by introduction of functional groups, oxidation, reduction, substitution of atoms, etc., to the extent that the structure and properties of the parent compound are not significantly changed.

As used herein, the term "comprising as an effective ingredient" means that the composition according to an embodiment of the present disclosure comprises 3-hydroxypropionic acid or a derivative thereof in an amount sufficient to achieve an effect for preventing, alleviating or ameliorating alopecia.

As a result of continuous research, the present inventors have found that 3-hydroxypropionic acid can exhibit excellent effects for preventing, alleviating, or ameliorating alopecia.

In particular, 3-hydroxypropionic acid not only inhibits the action of TGF-$\beta$1 factor, but also has an inhibitory effect on 5$\alpha$-reductase, whereby a cosmetic composition comprising 3-hydroxypropionic acid or a derivative thereof as an effective ingredient can exhibit excellent effects for preventing, alleviating, or ameliorating alopecia.

According to an embodiment of the present disclosure, a cosmetic composition for preventing, alleviating or ameliorating alopecia comprising 3-hydroxypropionic acid or a derivative thereof as an effective ingredient is provided.

The cosmetic composition for preventing, alleviating or ameliorating alopecia comprises 3-hydroxypropionic acid or a derivative thereof as an effective ingredient. Preferably, the cosmetic composition for preventing, alleviating or ameliorating alopecia comprises 3-hydroxypropionic acid as an effective ingredient.

3-Hydroxypropionic acid synthesized by a chemical method or a biological method can be applied as an active ingredient.

According to an embodiment, 3-hydroxypropionic acid obtained through a biological process such as fermenting a strain having 3-hydroxypropionic acid production ability can be preferably used as the effective ingredient. The 3-hydroxypropionic acid obtained by the above method can include a radioactive carbon isotope ($^{14}$C).

Preferably, the effective ingredient may have a radioactive carbon isotope content of 20 pMC(percent modern carbon) or more and a biocarbon content of 20 wt.% or more as measured according to ASTM D6866-21 standard. Specifically, the effective ingredient may have a radioactive carbon isotope content of 20 pMC or more, or 50 pMC or more, or 90 pMC or more, or 100 pMC or more, or 90 to 110 pMC and a biocarbon content of 20 wt.% or more, or 50 wt.% or more, or 80 wt.% or more, or 90 wt.% or more, or 95 wt.% or more, or 90 to 100 wt.%, as measured according to ASTM D6866-21 standard.

The radioactive carbon isotope content (pMC) refers to a ratio of the radioactive carbon isotope($^{14}$C) contained in 3-hydroxypropionic acid to the radioactive carbon isotope($^{14}$C) of the modern reference material. For reference, the effectiveness of a nuclear test program in the 1950s is continuing and not extinguished, and thus the radioactive carbon isotope content (pMC) may be greater than 100%.

The biocarbon content represents the content of biocarbon with respect to a total carbon content included in 3-hydroxypropionic acid, which may mean that a higher value can correspond to an eco-friendly compound.

If the radioactive carbon isotope content (pMC) and biocarbon content of the 3-hydroxypropionic acid are too low, eco-friendliness can be reduced and it may not be regarded as a bio-derived material.

The radioactive isotope ($^{14}C$) can be nearly one per $1 \times 10^{12}$ carbon atoms in the earth's atmosphere and have a half-life of about 5,700 years, and it may be abundant in an upper atmosphere due to a nuclear reaction involving cosmic rays and common nitrogen ($^{14}N$).

In fossil fuels, radioactive isotopes have decayed long ago and thus a $^{14}C$ ratio can be substantially zero. When bio-derived materials are used as a raw material for 3-hydroxypropionic acid or when fossil fuels are used together, a content of radioactive carbon isotopes (pMC) contained in 3-hydroxypropionic acid and a content of biocarbon can be measured according to ASTM D6866-21 standard.

For example, carbon atoms included in the compound to be measured can be made into the form of graphite or carbon dioxide gas, and then measured with a mass spectrometer, or according to a liquid scintillation spectroscopy. In this case, two isotopes can be separated using an accelerator for separating $^{14}C$ ions from $^{12}C$ ions, and thus the content and content ratio can be measured with a mass spectrometer.

The cosmetic composition can inhibit the action of TGF-β1 factor secreted from hair follicle cells. Thereby, the cosmetic composition can inhibit the promotion of the anagen of the hair follicle into the catagen, thereby exhibiting an effect for preventing, alleviating or ameliorating alopecia.

Further, the cosmetic composition can inhibit the activity of 5α-reductase which produces dihydrotestosterone by the action of the effective ingredient. Thereby, the cosmetic composition can prevent the production of dihydrotestosterone, which inhibits cell division in hair follicles and inhibits hair growth, thereby exhibiting an effect for preventing, alleviating or ameliorating alopecia.

According to an embodiment, the cosmetic composition can contain 0.01 wt.% to 1.5 wt.% of the effective ingredient based on the total weight of the cosmetic composition.

Specifically, the cosmetic composition can contain 0.01 wt.% or more or 0.012 wt.% or more; and 1.5 wt.% or less, or 1.25 wt.% or less, or 1.1 wt.% or less, or 1.0 wt.% or less of the effective ingredient based on the total weight of the cosmetic composition.

In order to ensure that the effect for preventing, alleviating or ameliorating alopecia can be sufficiently achieved by the action of the effective ingredient, the cosmetic composition preferably contains 0.01 wt.% or more or 0.012 wt.% or more of the effective ingredient.

However, if the effective ingredient is contained in an excessive amount, the effect for preventing, alleviating or ameliorating alopecia may be rather reduced or side effects such as cytotoxicity may appear. Therefore, the cosmetic composition preferably contains 1.5 wt.% or less, or 1.25 wt.% or less, or 1.1 wt.% or less, or 1.0 wt.% of the effective ingredient.

Preferably, the cosmetic composition may contain 0.01 wt.% to 1.5 wt.%, or 0.01 wt.% to 1.25 wt.%, or 0.01 wt.% to 1.1 wt.%, or 0.01 wt.% to 1.0 wt.%, or 0.012 wt.% to 1.0 wt.% of the effective ingredient based on the total weight of the cosmetic composition.

According to an embodiment, the cosmetic composition may have a conventional cosmetic formulation in the technical field to which the present disclosure belongs. The cosmetic composition may be formulated in in a wide variety of forms, for example, including a solution, an emulsion, a suspension, a paste, a gel, a cream, a lotion, a powder, a soap, surfactant-containing cleanser, an oil, a powder foundation, an emulsion foundation, a wax foundation, a spray, and the like.

The cosmetic composition may further comprise a physiologically acceptable medium comprising water or a mixture of water and an organic solvent.

As an example, the organic solvent may include C1-C4 lower alcohols such as acetone, ethanol and isopropyl alcohol, alkylene glycols such as ethylene glycol and propylene glycol, ethylene glycol monomethyl ether, monoethyl ether, monobutyl ether, propylene glycol, and the like. The physiologically acceptable medium may be applied in an amount of 1 to 90 wt.% based on the total weight of the cosmetic composition.

The medium can be thickened using a thickener commonly used in cosmetics or pharmaceuticals. The thickener can be applied in an amount of 0.1 to 5 wt.% based on the total weight of the composition.

In addition, according to some embodiments of the cosmetic composition, a person skilled in the art can select adjuvants that are generally required for preparing such compositions. As an example, the adjuvants may comprise preservatives, stabilizers, pH regulators, osmotic pressure modifiers, emulsifiers, sunscreens, antioxidants, fragrances, dyes, anionic surfactants, cationic surfactants, nonionic surfactants, and amphoteric surfactants.

According to an embodiment, when the formulation of the cosmetic composition is a powder or spray, it may comprise carrier components such as lactose, talc, silica, aluminum hydroxide, calcium silicate, polyamide powder, and the like.

According to an embodiment, when the formulation of the cosmetic composition is a paste, gel, or cream, it may comprise carrier components such as animal fats, vegetable oils, waxes, paraffins, starch, tragacanth, cellulose derivatives, polyethylene glycols, silicones, bentonite, silica, talc, zinc oxide, and the like.

According to an embodiment, when the formulation of the cosmetic composition is a solution and emulsion, it may comprise carrier components such as solvent, solubilizer and emulsifier. For example, the formulation of solutions and emulsions may comprise water, ethanol, isopropanol, ethyl carbonate, ethyl acetate, benzyl alcohol, benzyl benzoate, propylene glycol, 1.3-butyleneglycol oils, glycerol fatty acid ester, polyethylene glycol or fatty acid ester of sorbitan, and the like.

According to an embodiment, when the formulation of the cosmetic composition is a suspension, it may comprise liquid diluents such as water, ethanol or propylene glycol; suspending agents such as ethoxylated isostearyl alcohols, polyoxyethylene sorbitol esters and polyoxy ethylene sorbitan esters; microcrystalline cellulose, aluminum metahydroxide, bentonite, and the like.

According to an embodiment, when the formulation of the cosmetic composition is a cleansing agent containing a surfactant, it may comprise carrier components such as an aliphatic alcohol sulfate, aliphatic alcohol ether sulfate, sulfosuccinic acid monoester, isethionate, imidazolinium derivative, sodium methyl cocoyl taurate, sodium lauroyl sarcosinate, fatty acid amide ether sulfate, alkylamidobetaine, fatty alcohol, fatty acid glyceride, fatty acid diethanolamide, vegetable oil, lanolin derivative, ethoxylated glycerol fatty acid ester, and the like.

According to an embodiment, the cosmetic composition may be intended for hair or scalp use.

For example, the cosmetic composition may have a formulation that include a hair shampoo, a hair rinse, a hair tonic, a hair treatment, a scalp treatment, a hair lotion, a hair cream, a hair spray, a hair form, a hair gel, a hair pack, a hair aerosol, a hair dye, a balm, a powder, a solid soap, or a liquid soap.

According to another embodiment of the present disclosure, there is provided use of 3-hydroxypropionic acid or a derivative thereof for the preparation of cosmetics for preventing, alleviating or ameliorating alopecia.

Preferably, the use of 3-hydroxypropionic acid or a derivative thereof for the preparation of the above-mentioned cosmetic composition for preventing, alleviating or ameliorating alopecia is provided.

3-Hydroxypropionic acid inhibits the action of TGF-$\beta$1 factor and has an inhibitory effect on 5$\alpha$-reductase, whereby a cosmetic composition comprising 3-hydroxypropionic acid or a derivative thereof as an effective ingredient can be used for preventing, alleviating or ameliorating alopecia.

In the above applications, the effective ingredient, i.e., 3-hydroxypropionic acid or a derivative thereof, can be used in an amount of 0.01 wt.% to 1.5 wt.% based on the total weight of the cosmetic composition.

In order to ensure that the effect for preventing, alleviating or ameliorating alopecia can be sufficiently achieved by the action of the active ingredient, the effective ingredient is preferably used in an amount of 0.01 wt.% or more or 0.012 wt.% or more based on the total weight of the cosmetic composition. However, if the effective ingredient is used in an excessive amount, the above-mentioned effects may be rather reduced or side effects such as cytotoxicity may appear. Therefore, the effective ingredient is preferably used in an amount of 1.5 wt.% or less, or 1.25 wt.% or less, or 1.1 wt.% or less, or 1.0 wt.% or less, based on the total weight of the cosmetic composition. Preferably, the effective ingredient may be used in an amount of 0.01 wt.% to 1.5 wt.%, or 0.01 wt.% to 1.25 wt.%, or 0.01 wt.% to 1.1 wt.%, or 0.01 wt.% to 1.0 wt.%, or 0.012 wt.% to 1.0 wt.%, based on the total weight of the cosmetic composition.

According to yet another embodiment of the present disclosure, there is provided a method of preventing, alleviating or ameliorating alopecia by using a cosmetic composition comprising 3-hydroxypropionic acid or a derivative thereof as an active ingredient. Specifically, a method of inhibiting the action of TGF-$\beta$1 factor secreted from hair follicle cells is provided. In addition, a method of inhibiting the activity of 5$\alpha$-reductase which produces dihydrotestosterone by using a cosmetic composition comprising 3-hydroxypropionic acid or a derivative thereof as an active ingredient is provided.

Preferably, the method of preventing, alleviating or ameliorating alopecia can be performed by using the above-mentioned cosmetic composition for preventing, alleviating or ameliorating alopecia. The method can be performed with an application frequency of one to several times a day (for example, one to five times or one to three times) for one to six months, one to seven days a week. The method can be performed by applying the cosmetic composition to a human body site (e.g., hair or scalp) in need of the prevention, alleviation or amelioration of alopecia. Specifically, there may be mentioned a method of washing a hair using the cosmetic composition in the form of a hair shampoo formulation, a method of washing or massaging a scalp using the cosmetic composition in the form of a scalp treatment formulation, or a method of applying the cosmetic composition in the form of a balm preparation to the scalp, etc.

According to yet another embodiment of the present disclosure, there is provided a pharmaceutical composition for preventing or treating alopecia comprising 3-hydroxypropionic acid or a derivative thereof as an active ingredient.

As a result of continuous research, the present inventors have found that 3-hydroxypropionic acid can exhibit excellent effects for preventing or treating alopecia.

In particular, 3-hydroxypropionic acid not only inhibits the action of TGF-$\beta$1 factor, but also has an inhibitory effect on 5$\alpha$-reductase, whereby a pharmaceutical composition comprising 3-hydroxypropionic acid or a derivative thereof as an active ingredient can exhibit excellent effects for preventing or treating alopecia.

The pharmaceutical composition for preventing or treating alopecia comprises 3-hydroxypropionic acid or a derivative thereof as an active ingredient. Preferably, the pharmaceutical composition for preventing or treating alopecia comprises 3-hydroxypropionic acid as an active ingredient.

3-Hydroxypropionic acid synthesized by a chemical method or a biological method can be applied as an active ingredient.

According to an embodiment, the effective ingredient may be preferably 3-hydroxypropionic acid obtained through a biological process such as fermenting a strain having 3-hydroxypropionic acid production ability. The 3-hydroxypropionic acid obtained by the above method may include a radioactive carbon isotope ($^{14}C$).

Preferably, the active ingredient may have a radioactive carbon isotope content of 20 pMC (percent modern carbon) or more and a biocarbon content of 20 wt.% or more as measured according to ASTM D6866-21 standard. Specifically, the active ingredient may have a radioactive carbon isotope content of 20 pMC or more, or 50 pMC or more, or 90 pMC or more, or 100 pMC or more, or 90 to 110 pMC, and a biocarbon content of 20 wt.% or more, or 50 wt.% or more, or 80 wt.% or more, or 90 wt.% or more, or 95 wt.% or more, or 90 to 100 wt.%, as measured in accordance with ASTM D6866-21 standard.

The pharmaceutical composition for preventing or treating alopecia can inhibit the action of TGF-β1 factor secreted from hair follicle cells due to the action of the effective ingredient. Thereby, the pharmaceutical composition can inhibit the promotion of the anagen of the hair follicle into the catagen, thereby exhibiting an effect for preventing, alleviating or ameliorating alopecia.

Further, the pharmaceutical composition for preventing or treating alopecia can inhibit the activity of 5α-reductase which produces dihydrotestosterone by the action of the effective ingredient. Thereby, the pharmaceutical composition can prevent the production of dihydrotestosterone, which inhibits cell division in hair follicles and inhibits hair growth, thereby exhibiting an effect for preventing, alleviating or ameliorating alopecia.

According to an embodiment, the pharmaceutical composition can contain 0.01 wt.% to 1.5 wt.% of the effective ingredient based on the total weight of the pharmaceutical composition.

Specifically, the pharmaceutical composition can contain 0.01 wt.% or more or 0.012 wt.% or more; and 1.5 wt.% or less, or 1.25 wt.% or less, or 1.1 wt.% or less, or 1.0 wt.% or less of the effective ingredient based on the total weight of the pharmaceutical composition.

In order to ensure that the effect for preventing or treating alopecia can be sufficiently achieved by the action of the active ingredient, the pharmaceutical composition preferably contains 0.01 wt.% or more or 0.012 wt.% or more of the effective ingredient.

However, if the effective ingredient is included in an excessive amount, the effect for preventing or treating alopecia may be rather reduced or side effects such as cytotoxicity may appear. Therefore, the pharmaceutical composition preferably contains 1.5 wt.% or less, or 1.25 wt.% or less, or 1.1 wt.% or less, or 1.0 wt.% or less of the effective ingredient.

Preferably, the pharmaceutical composition may comprise 0.01 wt.% to 1.5 wt.%, or 0.01 wt.% to 1.25 wt.%, or 0.01 wt.% to 1.1 wt.%, or 0.01 wt.% to 1.0 wt.%, or 0.012 wt.% to 1.0 wt.% of the effective ingredient based on the total weight of the composition.

The pharmaceutical composition may further comprise a pharmaceutically acceptable carrier.

As the carrier, those commonly used in the preparation of the pharmaceutical composition may be applied without particular limitation. As an example, the carrier may be sucrose, sorbitol, mannitol, lactose, dextrose, starch, gum acacia, calcium phosphate, alginate, gelatin, calcium silicate, microcrystalline cellulose, polyvinylpyrrolidone, cellulose, water, syrup, methyl cellulose, propyl 4-hydroxybenzoate, talc, magnesium stearate, mineral oil, and the like. Pharmaceutically acceptable carriers and formulations are described in detail in Remington's The Science and Practice of Pharmacy (23rd Edition, 2020).

The pharmaceutical composition may be orally or parenterally administered. In case of the parenteral administration, this may be administered through an application to skin, an intravenous injection, a subcutaneous injection, an intramuscular injection, an intraperitoneal injection, a dermal administration, etc.

An appropriate dosage of the pharmaceutical composition may be determined in comprehensive consideration of factors such as a patient's age, weight, gender, a formulation method, an administration method, administration route, and administration time.

The pharmaceutical composition can be formulated by applying a pharmaceutically acceptable carrier and/or excipient. The formulation of the pharmaceutical composition may be a solution, suspension, emulsion, ointment, powder, granule, tablet, or capsule in an aqueous medium or oil.

According to yet another embodiment of the present disclosure, there is provided use of 3-hydroxypropionic acid or a derivative thereof for the preparation of medicines for preventing or treating alopecia.

Preferably, the use of 3-hydroxypropionic acid or a derivative thereof for the preparation of the pharmaceutical composition for preventing or treating alopecia is provided.

3-Hydroxypropionic acid not only inhibits the action of TGF-β1 factor, but also has an inhibitory effect on 5α-reductase, whereby a pharmaceutical composition comprising 3-hydroxypropionic acid or a derivative thereof as an active ingredient can be used for preventing or treating alopecia.

In the above applications, the effective ingredient, i.e., 3-hydroxypropionic acid or a derivative thereof, can be used in an amount of 0.01 wt.% to 1.5 wt.% based on the total weight of the pharmaceutical composition.

In order to ensure that the effect for preventing or treating alopecia can be sufficiently achieved by the action of the active ingredient, the effective ingredient is preferably used in an amount of 0.01 wt.% or more or 0.012 wt.% or more based on the total weight of the pharmaceutical composition. However, if the effective ingredient is used in an excessive amount, the above-mentioned effects may be rather reduced or side effects such as cytotoxicity may appear. Therefore, the effective ingredient is preferably used in an amount of 1.5 wt.% or less, or 1.25 wt.% or less, or 1.1 wt.% or less, or 1.0 wt.% or less, based on the total weight of the pharmaceutical composition. Preferably, the effective ingredient may be used in an amount of 0.01 wt.% to 1.5 wt.%, or 0.01 wt.% to 1.25 wt.%, or 0.01 wt.% to 1.1 wt.%, or 0.01 wt.% to 1.0 wt.%, or 0.012 wt.% to 1.0 wt.%, based on the total weight of the pharmaceutical composition.

According to yet another embodiment of the present disclosure, there is provided a method of preventing or treating alopecia by using a pharmaceutical composition comprising 3-hydroxypropionic acid or a derivative thereof as an effective ingredient. Specifically, a method of inhibiting the action of TGF-β1 factor secreted from hair follicle cells by using a pharmaceutical composition comprising 3-hydroxypropionic acid or a derivative thereof as an active ingredient is provided. In addition, a method of inhibiting the activity of 5α-reductase which produces dihydrotestosterone by using a pharmaceutical composition comprising 3-hydroxypropionic acid or a derivative thereof as an active ingredient is provided.

Preferably, the method of preventing or treating alopecia can be performed by using the above-mentioned pharmaceutical composition for preventing or treating alopecia. The method can be performed by directly or indirectly applying the composition to a human body site (e.g., scalp) in need of the prevention or treatment of alopecia. The method can be performed orally or parenterally. The method can be performed with an application frequency of one to several times a day (for example, one to five times or one to three times) for one to six months, one to seven days a week. The method may be performed according to a conventional procedure in comprehensive consideration of factors such as a patient's age, weight, gender, formulation of 3-hydroxypropionic acid or a derivative thereof, an administration method, and administration time.

### [ADVANTAGEOUS EFFECTS]

According to the present disclosure, a cosmetic composition capable of exhibiting an excellent effect for preventing, alleviating or ameliorating alopecia and a pharmaceutical composition capable of exhibiting an excellent effect for preventing or treating alopecia are provided by not only inhibiting the action of the TGF-β1 factor but also having an inhibitory effect on 5α-reductase.

### [BRIEF DESCRIPTION OF THE DRAWINGS]

FIG. 1 is a graph showing the results of evaluating the ratio of hair follicle telogen according to Experimental Example 4.

FIG. 2 is a graph showing the results of evaluating the expression level of hair follicle cell proliferation-related factor protein according to Experimental Example 4.

### [DETAILED DESCRIPTION OF THE EMBODIMENTS]

Hereinafter, actions and effects of the invention will be described in more detail with reference to specific examples. However, the following examples are provided to assist the understanding of the invention, and are not intended to limit the scope of the invention in any way. It would be obvious to those skilled in the art that various changes and modifications can be made without departing from the sprit and scope of the invention.

Preparation Example 1

Prepared was a recombinant vector incorporating a gene encoding glycerol dehydratase and aldehyde dehydrogenase, which are known to produce 3-hydroxypropionic acid using glycerol as a substrate. The prepared recombinant vector was introduced into the *E. coli* W3110 strain to prepare a 3-hydroxypropionic acid-producing strain.

Specifically, pCDF_J23101_dhaB_gdrAB_J23100_aldH_btuR vector, was obtained by cloning BtuR gene encoding adenosyltransferase into plasmid pCDF including a gene(dhaB) encoding glycerol dehydratase, a gene(aldH) encoding aldehyde dehydrogenase and a gene(gdrAB) encoding glycerol dehydratase reactivase. The vector was introduced into W3110 strain (KCCM 40219) by electroporation using an electroporation device (Bio-Rad, Gene Pulser Xcell) to prepare a 3-hydroxypropionic acid-producing strain.

The 3-hydroxypropionic acid-producing strain was fermented and cultured at 35°C in a 5 L fermenter by using unpurified glycerol as a carbon source, so as to produce 3-hydroxypropionic acid. In order to prevent a decrease in pH due to the production of 3-hydroxypropionic acid, calcium hydroxide ($Ca(OH)_2$), an alkali metal salt, was added to maintain a neutral pH during fermentation.

After fermentation culture, cells were removed by centrifugation (4000 rpm, 10 minutes, 4°C), and primary purification was performed by using activated carbon. The concentration of 3-hydroxypropionic acid in the fermented solution obtained after the primary purification was at a level of 50 to 100 g/L, and the fermented solution was concentrated at a concentration of 600 g/L using a rotary evaporator (50°C, 50 mbar) to prepare a concentrate, which was then stirred at room temperature (300 rpm) to produce $Ca(3HP)_2$ crystals.

The resulting crystals were washed three times with ethanol and dried in an oven at 50°C to finally recover crystals. The weight of the crystals was measured, the crystals were dissolved in water to obtain 3-hydroxypropionic acid(3HP) using high performance liquid chromatography(HPLC).

Preparation Example 2

3-Hydroxypropionic acid(3HP) was obtained in substantially the same manner as in Preparation Example 1, except for using $Mg(OH)_2$ instead of $Ca(OH)_2$ as an alkali metal salt.

Example 1

0.025 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a hair lotion.

Example 2

0.05 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a hair lotion.

Example 3

0.0125 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a hair tonic.

Example 4

0.025 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a hair tonic.

Example 5

0.05 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a hair tonic.

Example 6

0.025 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a pharmaceutical composition as an ointment formulation.

Example 7

0.05 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a pharmaceutical composition as an ointment formulation

Example 8

0.0125 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a pharmaceutical composition as an emulsion formulation.

Example 9

0.025 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a pharmaceutical composition as an emulsion formulation.

Example 10

0.05 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a pharmaceutical composition as an emulsion formulation.

Experimental Example 1

(Measurement of radioactive carbon isotope and biocarbon content)

The radioactive carbon isotope content(pMC) and biocarbon content of the 3-hydroxypropionic acid obtained from Preparation Examples 1 and 2 were measured according to ASTM D6866-21 (Method B).

The biocarbon content means the content of biocarbon contained in the 3-hydroxypropionate crystals obtained from Preparation Examples 1 and 2, and the radioactive carbon isotope ratio(pMC) refers to a ratio of the radioactive carbon isotope ($^{14}C$) contained in the 3-hydroxypropionate crystals to the radioactive carbon isotope ($^{14}C$) of the modern reference material.

[Table 1]

| | Concentration (g/L) | Radioactive carbon isotope ratio (pMC) | Biocarbon content (wt.%) |
|---|---|---|---|
| Preparation Example 1 | 600 | 101.52 | 100 |
| Preparation Example 2 | 800 | 102.23 | 100 |

Referring to Table 1, it was confirmed that both of the 3HP salt crystals of Preparation Examples 1 and 2 have a radioactive carbon isotope ratio of 101 pMC or more and a biocarbon content of 100 wt.%.

Experimental Example 2

Human follicle dermal papilla cells (PromoCell GmbH) were cultured in human follicle dermal papilla cells growth medium(PromoCell GmbH) supplemented with fetal calf serum(FCS), bovine pituitary extract(BPE), basic fibroblast growth factor(bFGF), and insulin at 37°C and 5% $CO_2$.

The cultured human follicle dermal papilla cells were divided into $5 \times 10^3$ cells in a 96-well plate, and when the cells reached 80% or more confluency, they were treated with the 3-hydroxypropionic acid aqueous solution according to Preparation Example 1 at four concentrations (3HP concentrations: 0 wt.%, 0.0125 wt.%, 0.025 wt.%, and 0.05 wt.%), and then cultured for 24 hours. WST substrate solution (high sensitivity water soluble tetrazolium salt, CCK-8, DOJINDO) was then added to the medium, and cultured at 37°C for 2 hours.

During the culture, a water-insoluble formazan was produced by the reaction between WST and intracellular dehydrogenase, and the absorbance (OD, wavelength 450 nm) was measured. The cell viability was calculated according to the following Equation 1 below, and the results are shown in Table 1 below.

$$\text{Cell viability(\%)} = [(OD_{\text{experimental group}} - OD_{\text{control group}}) / (OD_{\text{negative control group}} - OD_{\text{control group}})] \times 100 \qquad \text{[Equation 1]}$$

In Equation 1, the 'experimental group' is the group treated with an aqueous solution containing 3HP; the 'control group' is the medium containing only the WST substrate; and the 'negative control group' is the group in which a medium containing WST substrate was treated with an aqueous solution of 0% 3HP.

[Table 2]

| | Human follicle dermal papilla cell viability (%) | | | | |
|---|---|---|---|---|---|
| 3HP treated concentration | 0 % (negative control group) | 0.0125 % | 0.025 % | 0.05 % | 0.1% |
| Once | 100.000 | 90.844 | 86.624 | 86.465 | 77.548 |
| Two times | 100.000 | 92.037 | 84.153 | 86.027 | 72.756 |
| Three times | 100.000 | 95.062 | 89.275 | 85.031 | 75.694 |
| Average | 100.000 | 92.648 | 86.684 | 85.841 | 75.333 |
| Standard deviation | 0.000 | 2.174 | 2.561 | 0.735 | 2.416 |
| Significance probability relative to negative control group (p-value) | - | 0.028 | 0.012 | 0.001 | 0.003 |

The experimental results showed that the cell viability was high under the above treatment concentrations of 3-hydroxypropionic acid.

Experimental Example 3

Human follicle dermal papilla cells (PromoCell GmbH) were cultured in human follicle dermal papilla cells growth medium(PromoCell GmbH) supplemented with fetal calf serum(FCS), bovine pituitary extract(BPE), basic fibroblast growth factor(bFGF), and insulin at 37°C and 5% $CO_2$.

The cultured human follicle dermal papilla cells were divided into $5 \times 10^4$ cells in a 96-well plate, and when the cells reached 80% or more confluency, they were treated with the 3-hydroxypropionic acid aqueous solution according to Preparation Example 1 at four concentrations (3HP concentrations: 0 wt.%, 0.0125 wt.%, 0.025 wt.%, and 0.05 wt.%), and then cultured for 24 hours. Then, the total RNA in the cells was extracted, and cDNA was synthesized from the extracted RNA. The synthesized cDNA was subjected to real-time polymerase chain reaction. And, the relative quantitative analysis of each gene was performed using GAPDH (one of the house-keeping genes). The relative quantification (RQ) value was calculated according to the following Equation 2.

[Equation 2]

$$RQ = 2 - \triangle\triangle C_T$$

$$\triangle\triangle C_T = \triangle C_T(\text{Treatment}) - \triangle C_T(\text{Control})$$

$$\triangle C_T = C_T(\text{Target gene}) - C_T(\text{House-keeping gene})$$

In the Equation 2, 'Treatment' is the group treated with 3HP; 'Control' is the group not treated with 3HP; 'Target gene' is Srd5$\alpha$1 and TGF-$\beta$1, and 'House-keeping gene' is GAPDH.

[Table 3]

| | 5$\alpha$-reductase gene expression level (RQ) | | | |
|---|---|---|---|---|
| 3HP treated concentration | 0% | 0.0125 % | 0.025 % | 0.05 % |
| Once | 0.982 | 0.856 | 0.871 | 1.322 |
| Two times | 0.992 | 0.815 | 0.880 | 1.133 |
| Three times | 1.026 | 0.927 | 0.914 | 1.347 |
| Average | 1.000 | 0.866 | 0.888 | 1.267 |
| Standard deviation | 0.023 | 0.057 | 0.023 | 0.117 |

(continued)

|  | 5α-reductase gene expression level (RQ) | | |
|---|---|---|---|
| 3HP treated concentration | 0% | 0.0125 % | 0.025 % | 0.05 % |
| p-value | - | 0.028 | 0.000 | 0.052 |

[Table 4]

|  | TGF-β1 gene expression level (RQ) | | |
|---|---|---|---|
| 3HP treated concentration | 0% | 0.0125 % | 0.025 % | 0.05 % |
| Once | 1.006 | 0.756 | 0.704 | 1.314 |
| Two times | 1.049 | 0.864 | 0.723 | 1.400 |
| Three times | 0.944 | 0.785 | 0.655 | 1.182 |
| Average | 1.000 | 0.802 | 0.694 | 1.298 |
| Standard deviation | 0.053 | 0.056 | 0.035 | 0.110 |
| p-value | - | 0.018 | 0.001 | 0.012 |

Referring to the experimental results, it was confirmed that the expression of the TGF-β1 gene and the 5α-reductase gene could be significantly inhibited under the treatment concentration of the 3-hydroxypropionic acid.

Experimental Example 4

The ratio of hair follicle telogen and the expression level of hair follicle cell proliferation-related factor proteins depending on whether 3-hydroxypropionic acid was applied or not were evaluated according to the following methods.

(1) Tissue culture conditions

The human hair follicles or scalp tissues provided for research purposes (IRB No. 4-2023-1052; Institutional Review Board, Severance Medical Center, Yonsei University College of Medicine) were washed several times with PBS to remove residual impurities, and randomly divided into each group. The hair follicles and scalp tissues were cultured in Williams' Medium E (Sigma) supplemented with 2 mM L-glutamine (Sigma-Aldrich), 10 μg/ml insulin (Sigma-Aldrich), 100 ng/ml hydrocortisone (Sigma-Aldrich), 0.1% fungizone (Gibco, USA), and 1% antibioticantimycotic (Gibco), and 1% penicillin-streptavidin (Gibco) at 37°C in 5% $CO_2$.

(2) Evaluation of the ratio of hair follicle telogen

To induce the telogen in the anagen hair follicle tissue, the test composition was treated with 1 μM dihydro-testosterone (DHT). The medium was replaced every 3 days and cultured for 6 days. The ratio of hair follicle telogen was visually evaluated based on the ratio of hair follicles in which the telogen occurred for 6 days compared to day 0.

(3) Evaluation of the expression level of cell proliferation-related factor proteins

After the scalp tissue was treated with the test composition for 24 hours, the obtained tissue was prepared into a frozen block, and frozen sectioned at a thickness of 5 μm using a Cryostat microtome (Leica Biosystems, Germany), and the tissue sections were attached onto Silane-coated slides. Then, in order to stain the tissue sections and photograph the cross sections, the OCT compound was removed, a primary antibody specifically binding to Ki67 (Anti-Ki67 antibody: ab15580; abcam) was attached, and a fluorescently labeled secondary antibody specifically binding to this primary antibody (Goat pAb to Rb IgG (H+L): a11012; Invitrogen) was attached. Finally, the tissues were fixed with a fixative containing blue fluorescent DAPI (VECTASHIELD® mounting medium with DAPI; Vector laboratories) that stains the cell nucleus, and then images were taken at 100x magnification using a fluorescence microscope (M2; Zeiss). The percentage of Ki67-expressing cells among the total cells labeled with DAPI was measured from the captured images, and a higher value means a higher number of Ki67-expressing cells.

[Table 5]

|  | Telogen ratio (%) |
|---|---|
| Control | 36.667 |
| DHT | 40.000 |
| DHT + 0.1% 3HP | 6.250 |
| DHT + 0.5% 3HP | 0.000 |
| DHT + 1.0% 3HP | 0.000 |
| DHT + 0.5 mM Finasteride | 0.000 |
| DHT + 2 ng/ml Biotin | 10.000 |

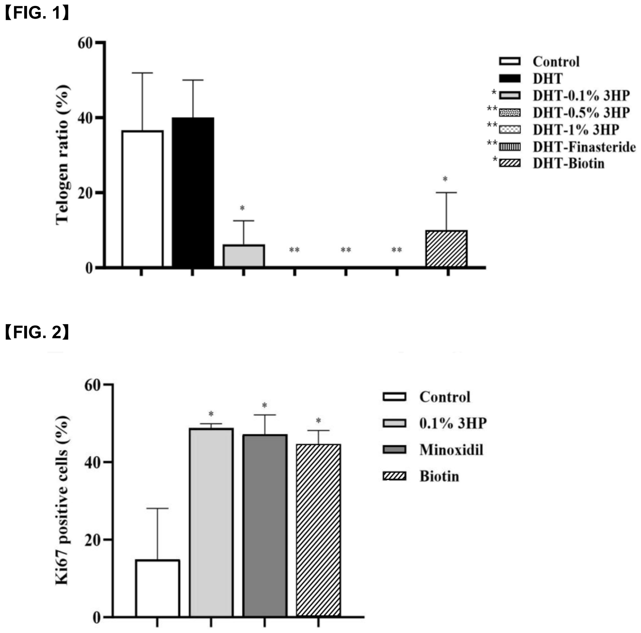

Referring to Table 5 and FIG. 1, it was confirmed that in the evaluation of the hair follicle telogen ratio, the hair follicle telogen ratio significantly decreased when 0.1%, 0.5%, and 1.0% 3HP were applied compared to the DHT control group.

[Table 6]

|  | Ki67 positive cells (%) |
|---|---|
| Control | 14.934 |
| 0.1% 3HP | 48.745 |
| 0.1 mM Minoxidil | 47.187 |
| 2 ng/ml Biotin | 44.631 |

Referring to Table 6 and FIG. 2, it was confirmed that in the evaluation of the proliferation of hair follicle cells through Ki67 immunofluorescence staining, the proliferation effect of hair follicle cells significantly increased when 0.1% 3HP was applied compared to the negative control group.

Formulation Example 1

0.2 wt.% of 3-hydroxypropionic acid obtained from Preparation Example 1, 3.0 wt.% of glycerin, 2.0 wt.% of butylene glycol, 2.0 wt.% of propylene glycol, 0.1 wt.% of polyacrylic acid, 0.2 wt.% of polyethylene glycol-12 nonylphenyl ether, 0.4 wt.% of polysorbate-80, 10.0 wt.% of ethanol, 0.1 wt.% of triethanolamine, and the remaining amount of purified water were mixed to prepare a hair tonic.

Formulation Example 2

1.0 wt.% of 3-hydroxypropionic acid obtained from Preparation Example 1, 3.0 wt.% of glycerin, 3.0 wt.% of butylene glycol, 7.0 wt.% of liquid paraffin, 7.0 wt.% of beta-glucan, 3.0 wt.% of caprylic/capric triglyceride, 5.0 wt.% of squalene, 1.5 wt.% of cetearyl glucoside, 1.2 wt.% of polysorbate-60, 0.4 wt.% of sorbitan stearate, 0.1 wt.% of polyacrylic acid, 0.1 wt.% of triethanol amine, and the remaining amount of purified water were mixed to prepare a hair lotion.

Formulation Example 3

1.0 wt.% of 3-hydroxypropionic acid obtained from Preparation Example 1, 0.5 wt.% of polyquaternium-10, 10 wt.% of sodium lauryl sulfate, 30 wt.% of polyoxyethylene lauryl ether, 5 wt.% of palm oil fatty acid diethanolamide, 0.5 wt.% of citric acid, 0.4 wt.% of fragrance, and the remaining amount of purified water are mixed to prepare a hair shampoo.

Formulation Example 4

1.0 wt.% of 3-hydroxypropionic acid obtained from Preparation Example 1, 1.0 wt.% of dye (Basic Brown 16), 4.5 wt.% of benzyl alcohol, 4.0 wt.% of glycerin, 5.0 wt.% of polyquaternium-6, and the remaining amount of a cream (a cream based on cetearyl alcohol) were mixed to prepare a hair dye.

Formulation Example 5

1.0 wt.% of 3-hydroxypropionic acid obtained from Preparation Example 1, 30 wt.% of methylpropanediol, 3 wt.% of stearamidopropyl dimethylamine, 2 wt,% of dipalmitoylethyl dimonium chloride, 5 wt.% of oil, 12 wt.% of cetearyl alcohol, and the remaining amount of purified water were mixed to prepare a hair treatment.

Formulation Example 6

1.0 wt.% of 3-hydroxypropionic acid obtained from Preparation Example 1, 0.006 wt.% of lecithin, 0.12 wt.% of amphoteric surfactant, 0.02 wt.% of nonionic surfactant, 2.5 wt.% of film forming polymer, 0.005 wt.% of cationic polymer, 3.0 wt.% of wetting agent, 2.0 wt.% of dimethicone, 5.0 wt.% of ethanol, 0.5 wt.% of pH regulator, and the remaining amount of purified water were mixed to prepare a hair spray.

Formulation Example 7

8 mg of 3-hydroxypropionic acid obtained from Preparation Example 1, 9 mg of vitamin C, 9 mg of vitamin E, 200 mg of lactose, and 200 mg of galactooligosaccharide were mixed, and granulated using a fluidized bed dryer, and 5 mg of sugar ester was added to produce a tablet composition. 500 mg of the tablet composition was tableted to prepare tablets.

Formulation Example 8

8 mg of 3-hydroxypropionic acid obtained from Preparation Example 1, 9 mg of vitamin C, 9 mg of vitamin E, 10 mg of vegetable hardened oil, and 9 mg of lecithin were mixed to prepare a soft capsule filling liquid. Separately, 65 wt.% of gelatin, 25 wt.% of glycerin, and 10 wt.% of sorbitol solution were mixed to prepare a soft capsule sheet. The soft capsule filling liquid was filled at 400 mg per capsule to prepare a soft capsule.

Formulation Example 9

8 mg of 3-hydroxypropionic acid obtained from Preparation Example 1, 9 mg of vitamin C, 9 mg of vitamin E, 500 mg of starch, and 200 mg of anhydrous crystalline glucose were mixed, formed into granules using a fluidized bed granulator, and then filled into a capsule to prepare a granule formulation.

Formulation Example 10

8 mg of 3-hydroxypropionic acid obtained from Preparation Example 1, 9 mg of vitamin C, 9 mg of vitamin E, 0.7 g of citric acid, 10 g of glucose, and 25 g of liquid oligosaccharide were mixed and then 300 ml of purified water were added to prepare a drink composition. The drink composition was filled into bottles at 200 ml each and sterilized at 130°C for about 5 seconds to prepare a drink formulation.

Formulation Example 11

30 mg of 3-hydroxypropionic acid obtained from Preparation Example 1, an appropriate amount of sterilized distilled water for injection, and an appropriate amount of a pH regulator were mixed to prepare an injection formulation.

While the present disclosure has been described with reference to limited embodiments, the present disclosure should be not limited to the disclosed embodiment and it will be apparent to those skilled in the art that various modifications and variations may be made without departing from the scope of the technical idea of the present disclosure and the scope of the appended claims and their equivalents.

**Claims**

1. A cosmetic composition for preventing, alleviating or ameliorating alopecia, comprising 3-hydroxypropionic acid or a derivative thereof as an effective ingredient.

2. The cosmetic composition for preventing, alleviating or ameliorating alopecia according to claim 1, wherein the cosmetic composition inhibits the action of TGF-$\beta$1 factor secreted from hair follicle cells.

3. The cosmetic composition for preventing, alleviating or ameliorating alopecia according to claim 1, wherein the cosmetic composition inhibits the activity of 5α-reductase which produces dihydrotestosterone.

4. The cosmetic composition for preventing, alleviating or ameliorating alopecia according to claim 1, wherein the cosmetic composition comprises 0.01 wt.% to 1 wt.% of the effective ingredient based on the total weight of the composition.

5. The cosmetic composition for preventing, alleviating or ameliorating alopecia according to claim 1, wherein the effective ingredient has a radioactive carbon isotope content of 20 pMC (percent modern carbon) or more and a biocarbon content of 20 wt.% or more as measured according to ASTM D6866-21 standard.

6. The cosmetic composition for preventing, alleviating or ameliorating alopecia according to claim 1, wherein the cosmetic composition further comprises a physiologically acceptable medium.

7. The cosmetic composition for preventing, alleviating or ameliorating alopecia according to claim 1, wherein the cosmetic composition is intended for hair or scalp use.

8. The cosmetic composition for preventing, alleviating or ameliorating alopecia according to claim 1, wherein the cosmetic composition has a formulation that includes a hair shampoo, a hair rinse, a hair tonic, a hair treatment, a scalp treatment, a hair lotion, a hair cream, a hair spray, a hair form, a hair gel, a hair pack, a hair aerosol, a hair dye, a balm, a powder, a solid soap, or a liquid soap.

9. A pharmaceutical composition for preventing or treating alopecia comprising 3-hydroxypropionic acid or a derivative thereof as an effective ingredient.

10. The pharmaceutical composition for preventing or treating alopecia according to claim 9, wherein the pharmaceutical composition inhibits the action of TGF-β1 factor secreted from hair follicle cells.

11. The pharmaceutical composition for preventing or treating alopecia according to claim 9, wherein the pharmaceutical composition inhibits the activity of 5α-reductase which produces dihydrotestosterone.

12. The pharmaceutical composition for preventing or treating alopecia according to claim 9, wherein the pharmaceutical composition comprises 0.01 wt.% to 1.5 wt.% of the effective ingredient based on the total weight of the composition.

13. The pharmaceutical composition for preventing or treating alopecia according to claim 9, wherein the effective ingredient has a radioactive carbon isotope content of 20 pMC (percent modern carbon) or more and a biocarbon content of 20 wt.% or more as measured according to ASTM D6866-21 standard.

14. The pharmaceutical composition for preventing or treating alopecia according to claim 9, wherein the pharmaceutical composition further comprises a pharmaceutically acceptable carrier.

15. The pharmaceutical composition for preventing or treating alopecia according to claim 9, wherein the pharmaceutical composition is for oral or parenteral use.

16. Use of 3-hydroxypropionic acid or a derivative thereof for the preparation of cosmetics for preventing, alleviating or ameliorating alopecia or medicines for preventing or treating alopecia.

17. A method of preventing, alleviating, ameliorating or treating alopecia by using a cosmetic composition or a pharmaceutical composition, comprising 3-hydroxypropionic acid or a derivative thereof as an active ingredient.

EP 4 684 783 A1

【FIG. 1】

【FIG. 2】

## INTERNATIONAL SEARCH REPORT

| International application No. |
| --- |
| **PCT/KR2024/096040** |

### A. CLASSIFICATION OF SUBJECT MATTER

**A61K 31/19**(2006.01)i; **A61P 17/14**(2006.01)i; **A61K 8/365**(2006.01)i; **A61Q 7/00**(2006.01)i; **A61Q 5/00**(2006.01)i; **A61K 31/191**(2006.01)i; **A61Q 9/04**(2006.01)i

According to International Patent Classification (IPC) or to both national classification and IPC

### B. FIELDS SEARCHED

Minimum documentation searched (classification system followed by classification symbols)

A61K 31/19(2006.01); A61K 31/185(2006.01); A61K 8/362(2006.01); A61K 8/365(2006.01); A61K 8/368(2006.01); A61K 8/44(2006.01); A61K 8/97(2006.01); A61K 8/9783(2017.01); A61Q 19/02(2006.01); A61Q 5/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3-하이드록시프로피온산(3-hydroxypropionic acid), 탈모(alopecia)

### C. DOCUMENTS CONSIDERED TO BE RELEVANT

| Category* | Citation of document, with indication, where appropriate, of the relevant passages | Relevant to claim No. |
| --- | --- | --- |
| A | US 2018-0271759 A1 (CONOPCO, INC. D/B/A UNILEVER) 27 September 2018 (2018-09-27)<br>See paragraph [0061]; and claim 1. | 1-16 |
| A | JP 5086539 B2 (LION CORP.) 28 November 2012 (2012-11-28)<br>See entire document. | 1-16 |
| A | KR 10-1695375 B1 (KOREA INSTITUTE OF OCEAN SCIENCE & TECHNOLOGY) 12 January 2017 (2017-01-12)<br>See entire document. | 1-16 |
| A | KR 10-2020-0113600 A (WI, Sam Gyul) 07 October 2020 (2020-10-07)<br>See entire document. | 1-16 |
| A | KR 10-2010-0060711 A (AMOREPACIFIC CORPORATION) 07 June 2010 (2010-06-07)<br>See entire document. | 1-16 |

☐ Further documents are listed in the continuation of Box C.　　☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **09 December 2024** | **09 December 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**EP 4 684 783 A1**

<table>
<tr><td><strong>INTERNATIONAL SEARCH REPORT</strong></td><td>International application No.<br><br><strong>PCT/KR2024/096040</strong></td></tr>
</table>

**Box No. II    Observations where certain claims were found unsearchable (Continuation of item 2 of first sheet)**

This international search report has not been established in respect of certain claims under Article 17(2)(a) for the following reasons:

1. ☑  Claims Nos.: **17**
   because they relate to subject matter not required to be searched by this Authority, namely:

   Claim 17 pertains to a method for treatment of the human body by surgery or therapy (PCT Article 17(2)(a)(i) and PCT Rule 39.1(iv)).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2024/096040**

| Patent document cited in search report | | | Publication date (day/month/year) | Patent family member(s) | | | Publication date (day/month/year) |
|---|---|---|---|---|---|---|---|
| US | 2018-0271759 | A1 | 27 September 2018 | CN | 107635541 | A | 26 January 2018 |
| | | | | CN | 110585051 | A | 20 December 2019 |
| | | | | CN | 110693732 | A | 17 January 2020 |
| | | | | EP | 3297600 | A1 | 28 March 2018 |
| | | | | EP | 3297600 | B1 | 30 January 2019 |
| | | | | JP | 2018-520088 | A | 26 July 2018 |
| | | | | JP | 6942635 | B2 | 29 September 2021 |
| | | | | WO | 2016-188691 | A1 | 01 December 2016 |
| JP | 5086539 | B2 | 28 November 2012 | JP | 2007-176826 | A | 12 July 2007 |
| KR | 10-1695375 | B1 | 12 January 2017 | None | | | |
| KR | 10-2020-0113600 | A | 07 October 2020 | KR | 10-2307509 | B1 | 29 September 2021 |
| KR | 10-2010-0060711 | A | 07 June 2010 | KR | 10-1017586 | B1 | 28 February 2011 |

Form PCT/ISA/210 (patent family annex) (July 2022)

**REFERENCES CITED IN THE DESCRIPTION**

**Patent documents cited in the description**

- KR 1020230120959
- KR 1020240099301
- KR 1020240099302